# FASCICULE DE BREVET EUROPEEN

(11) **EP 1 731 136 B1**
(45) Date de publication et mention de la délivrance du brevet: **15.10.2008**
(21) Numéro de dépôt: 06114333.5
(22) Date de dépôt: 22.05.2006
(51) Int. Cl.: A61K 8/89, A61K 8/92, A61Q 1/02, A61Q 1/06

(54) **Composition cosmétique contenant des portions de sphères creuses organosiliconées**
Kosmetische Zusammensetzung enthaltend Organosilikon-Hohlteilchen
Cosmetic composition containing hollow organosilicon particles

(30) Priorité: 09.06.2005 FR 0551553
(43) Date de publication de la demande: 13.12.2006
(73) Titulaire: L'ORÉAL, 75008 Paris (FR)
(72) Inventeur: Kani, Toshiyuki, Meguro-ku Tokyo, 152-0002 (JP); Dumousseaux, Christophe, Tokyo (JP)
(74) Mandataire: Boulard, Denis

(56) Documents cités:
- EP-A- 0 908 175
- EP-A- 1 530 961
- EP-A- 1 579 841
- PATENT ABSTRACTS OF JAPAN vol. 2003, no. 09, 3 septembre 2003 (2003-09-03) & JP 2003 128788 A (TAKEMOTO OIL & FAT CO LTD), 8 mai 2003 (2003-05-08)
- PATENT ABSTRACTS OF JAPAN vol. 2000, no. 10, 17 novembre 2000 (2000-11-17) & JP 2000 191789 A (TAKEMOTO OIL & FAT CO LTD), 11 juillet 2000 (2000-07-11)

## Description

La présente invention a pour objet une composition cosmétique destinée à être appliquée sur les matières kératiniques d'êtres humains, comme la peau, les lèvres, les cils, les sourcils, les ongles, les cheveux, et plus particulièrement sur la peau ou les lèvres.

La composition selon l'invention peut être une composition de maquillage ou de soin.

La composition peut être une composition de maquillage, en particulier un produit de maquillage des lèvres (rouge à lèvres), un fond de teint, un fard à paupières, un fard à joue, un produit anticernes, un eye-liner, un produit de maquillage du corps, un mascara, un vernis à ongles, un produit de maquillage des cheveux.

La composition peut être une composition de soin, en particulier un produit de soin de la peau du corps et du visage, notamment un produit solaire, un produit de coloration de la peau (tel qu'un autobronzant). La composition peut être également un produit capillaire, notamment pour le maintien de la coiffure ou la mise en forme des cheveux.

Les compositions cosmétiques contiennent souvent des agents épaississants pour conférer à la composition une viscosité adaptée à l'utilisation du produit. Dans le cas de compositions fluides, l'agent épaississant permet de conférer à la composition finale la viscosité souhaitée. Dans le cas de compositions sous forme de poudres, l'agent épaississant est utilisé pour conférer au liant les propriétés souhaitées : en effet, si le liant est trop fluide, la poudre obtenue présente un mauvais glissant à l'application.

L'agent épaississant peut être notamment introduit dans une phase grasse de la composition comprenant le plus souvent des huiles.

Il est connu, par exemple de la demande EP-A-908715, d'utiliser comme agents épaississants de milieu huileux, des élastomères de silicone qui confèrent à la composition des propriétés de texture et de douceur au toucher particulièrement intéressantes.

Toutefois, ces élastomères de silicone présentent cependant l'inconvénient d'être coûteux. Par ailleurs, pour pouvoir épaissir les huiles de manière satisfaisante, voir obtenir un gel macroscopique, il est nécessaire d'utiliser un taux élevé d'élastomères de silicones, soit au moins 20% en poids de matière active d'élastomères de silicones. En outre, les gels huileux obtenus avec ces élastomères peuvent conférer un toucher gras ou collant aux compositions.

Pour augmenter la viscosité d'une phase grasse liquide, il est par ailleurs connu d'introduire, dans des compositions cosmétiques, des particules particulières absorbant l'huile, en particulier des silices poreuses, comme décrit dans la demande FR 2856921. Cependant, après l'application de ces compositions sur la peau, le maquillage obtenu présente un effet desséchant dû à la présence des particules poreuses. Il en résulte une sensation d'inconfort pour l'utilisatrice notamment de tiraillement de la peau.

Il existe donc un besoin de disposer de compositions cosmétiques contenant un agent épaississant permettant d'épaissir la phase huileuse de la composition, et ne présentant pas d'effet desséchant après l'application sur la peau.

Il existe également un besoin de disposer de compositions cosmétiques présentant de bonnes propriétés d'étalement ou de glissant sur la peau ou les lèvres.

Les inventeurs ont mis en évidence qu'en associant, dans une phase huileuse, un élastomère de silicone non sphérique et des particules siliconées concaves, il était possible d'obtenir une viscosité élevée de la phase huileuse.
Par ailleurs, un tel mélange permet de conserver de bonnes propriétés cosmétiques, notamment des propriétés de non dessèchement, de non tiraillement, de douceur, de confort, de glissant, et d'étalement.

La présente invention a donc pour objet une composition comprenant une phase grasse comprenant au moins une huile, au moins un élastomère de silicone non sphérique, et des particules concaves d'un matériau siliconé, notamment sous forme de portions de sphères creuses. La composition est notamment une composition cosmétique ou dermatologique.

L'invention a également pour objet un procédé cosmétique (non thérapeutique) de maquillage ou de soin des matières kératiniques comprenant l'application sur lesdites matières kératiniques d'une composition cosmétique telle que définie précédemment. Les matières kératiniques sont notamment la peau et les lèvres.

L'invention a encore pour objet l'utilisation d'une composition cosmétique telle que définie précédemment pour obtenir un dépôt, notamment un maquillage, sur la peau ou les lèvres non desséchant et/ou ne tiraillant pas et/ou confortable.

### Elastomères de silicone

La composition selon l'invention comprend un élastomère de silicone non sphérique. L'élastomère de silicone peut être non émulsionnant ou émulsionnant.

La composition selon l'invention peut comprendre un élastomère de silicone non émulsionnant.

Le terme élastomères de silicone "non émulsionnant" définit des élastomères organopolysiloxane ne contenant pas de chaîne hydrophile telle que motifs polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.
Les organopolysiloxanes (A2) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).
Avantageusement, la somme du nombre de groupements éthyléniques par molécule du composé (A2) et le nombre d'atomes d'hydrogène liés au silicum par molécule du composé (B2) est d'au moins 4.
Le composé (B2) peut être sous toute structure moléculaire, notamment de structure chaîne linéaire, chaîne ramifiée, structure cyclique.
Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

L'élastomère de silicone non émulsionnant est généralement mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" par la société Shin Etsu, "DC 9040", "DC9041", "DC 9509", "DC9505", "DC 9506" par la société Dow Corning, "GRANSIL" par la société Grant Industries, "SFE 839" par la société General Electric.

Par élastomère de silicone émulsionnant on entend un élastomère de silicone comprenant au moins une chaîne hydrophile.

L'élastomère de silicone émulsionnant peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est véhiculé sous forme de gel dans au moins une huile hydrocarbonée et/ou une huile siliconée. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.
Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-21 0", "KSG-310", "KSG-320", "KSG-330", "KSG-340", "X-226146" par la société Shin Etsu, "DC9010", "DC9011" par la société Dow Corning.

L'élastomère de silicone émulsionnant peut être également choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ-₁ -O-[Gly]n-CₘH₂ₘ-₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet WO2004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

L'élastomère de silicone non sphérique peut être présent dans la composition selon l'invention en une teneur allant de 0,01 % à 30 % en poids, par rapport au poids total de la composition, de préférence allant de 0,1 % à 20 % en poids, et plus préférentiellement allant de 0,2 % à 10 % en poids.

### Les huiles

La composition selon l'invention comprend au moins une huile.

L'huile peut être choisie parmi les huiles volatiles, les huiles non volatiles, et leurs mélanges.

La composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

L'huile (ou le mélange d'huiles) peut être présente dans la composition selon l'invention en une teneur allant de 1 % à 80 % en poids, par rapport au poids total de la composition, de préférence allant de 5 % à 60 % en poids, de préférence allant de 5 % à 40 % en poids.

### Particules concaves siliconées

Les particules concaves siliconées présentes dans la composition selon l'invention sont en particulier des particules de portions de sphères creuses constituées d'un matériau siliconé.

Lesdites particules ont un diamètre moyen inférieur ou égal à 10 µm, notamment allant de 0,1 µm à 8 µm, préférentiellement de 0,2 à 7 µm, plus préférentiellement allant de 0,5 à 6 µm, et de préférence encore allant de 0,5 à 4 µm.

Par diamètre moyen, on entend la plus grande dimension de la particule. Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau organosiliconé est un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif peut être un groupe époxy, un groupe (méth)acryloxy, un groupe alkényle, un groupe mercaptoalkyle, aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.
Comme groupe (méth)acryloxy, on peut citer un groupe 3-méthacryloxypropyl, un groupe 3-acryloxypropyl.
Comme groupe alkényle, on peut citer un groupe vinyl, allyl, isopropényl.
Comme groupe mercaptoalkyle, on peut citer un groupe mercaptopropyl, mercaptoéthyl.
Comme groupe aminoalkyle, on peut citer un groupe 3-(2-aminoéthyl)aminopropyl, un groupe 3-aminopropyl, un groupe N,N-diméthylaminopropyl.
Comme groupe halogénoalkyle, on peut citer un groupe 3-chloropropyl, un groupe trifluoropropyl.
Comme groupe glycéroxy, on peut citer un groupe 3-glycéroxypropyl, un groupe 2-glycéroxyéthyl.
Comme groupe uréido, on peut citer un groupe 2-uréidoéthyl.
Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules d'organosilicone peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40°C et ne dépasse habituellement pas 30°C pour éviter une condensation prématurée des composés.

### Pour les groupements X et Y des composés (III) et (IV) :

Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant un groupement alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toulène sulfonique, l'acide dodécylbenzènesulfonique, l'acide dodécylsulfonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.
Des portions de sphères creuses en forme de fer à cheval sont aussi décrites dans la demande JP-A-2000-191789.

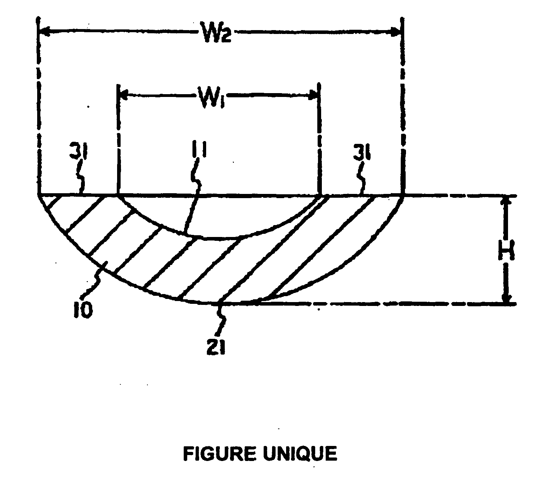

La figure annexée illustre une particule concave de portions de sphères en forme de bol en coupe transversale. La largeur W2 correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.
Les dimensions mentionnées ci-dessus sont obtenues en calculant la moyenne des dimensions de cent particules choisies sur une image obtenue au microscope électronique à balayage.

Comme particules concaves de portions de sphères utilisables selon l'invention, on peut citer :
- les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat)
- les particules constituées de l'organosilicone réticulé TAK-110 (polymère réticulé méthylsilanol/silicate) de la société TAKEMOTO OIL & FAT , en forme de bol , de largeur 0,8 µm, de hauteur 0,4 µm et d'épaisseur 130 nm (particules vendues sous la dénomination NLK-515 par la société Takemoto Oil & Fat)
- les particules constituées de l'organosilicone réticulé TAK-110 (polymère réticulé méthylsilanol/silicate) de la société TAKEMOTO OIL & FAT , en forme de bol , de largeur 7 µm, de hauteur 3,5 µm et d'épaisseur 200 nm (particules vendues sous la dénomination NLK-510 par la société Takemoto Oil & Fat)

Ces particules ont pour nom CTFA : methylesilanol/silicate crosspolymer.

Avantageusement, les particules concaves siliconées ont un diamètre moyen inférieur ou égal à 5 µm, notamment allant de 0,1 µm à 5 µm, préférentiellement allant de 0,2 à 5 µm, plus préférentiellement allant de 0,5 à 4 µm, et de préférence encore allant de 0,5 à 3 µm.

Ces particules de diamètre moyen inférieur ou égal à 5 µm permettent d'obtenir un épaississement de la phase grasse plus important que celui obtenu avec des particules de diamètre moyen supérieur à 5 µm. Ces particules permettent d'optimiser les propriétés de glissant, d'étalement, et de confort de la composition selon l'invention.

Les particules concaves siliconées peuvent être présentes dans la composition selon l'invention en une teneur allant de 0,01 % à 50 % en poids, par rapport au poids total de la composition, de préférence allant de 0,1 % à 30 % en poids, et préférentiellement allant de 1 % à 15 % en poids.

Selon un mode préféré de réalisation, le rapport massique de la teneur en particules concaves siliconées sur la teneur de l'élastomère de silicone va de 0,1 à 100, de préférence de 0,2 à 50, de façon encore plus préférentielle de 0,5 à 10.

Selon un mode particulier de réalisation, l'invention concerne une composition cosmétique comprenant :
i) des particules concaves siliconées ayant un diamètre moyen inférieur à 5 µm ;
ii) des particules d'au moins un élastomère de silicone non sphérique;
iii) au moins une huile ;

le rapport massique entre les particules concaves siliconées et les particules d'élastomère de silicone non sphérique est compris entre 0,1 et 100, de préférence entre 0,2 et 50, de façon encore plus préférentielle entre 0,5 et 10.

### Corps gras additionnel

Outre les huiles, la phase grasse de la composition selon l'invention peut comprendre des corps gras additionnels choisi parmi les cires, les corps gras pâteux, et leurs mélanges.

Par cire on entend un corps gras solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope. Elle peut être hydrocarbonée, fluorée et/ou siliconée et être d'origine animale, végétale, minérale ou synthétique.

La cire peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'α-oléfine, tel que les polymères PERFORMA V^{®} 825, 103 et 260, commercialisés par la société NEW PHASE TECHNOLOGIES ; les copolymères éthylène-propylène, tel que le PERFORMALENE^{®} EP 700, et les cires microcristallines dont le point de fusion est supérieur à 85 °C, tel que les HI-MIC^{®} 1070, 1080, 1090 et 3080, commercialisées par NIPPON SEIROU, et leurs mélanges.

La cire peut être présente dans la composition en une teneur allant de 0,01% à 20 %, en particulier de 1 à 15 %, plus particulièrement de 3 à 10 % en poids, par rapport au poids total de la composition.

Par "corps gras pâteux", on entend un composé gras à changement d'état solide/liquide réversible et comportant à la température de 23°C une fraction liquide et une fraction solide. On entend également par pâteux, le polylaurate de vinyle.
Le composé pâteux se présente avantageusement une dureté à 20 °C allant de 0,001 à 0,5 MPa, de préférence de 0,002 à 0,4 MPa.

Parmi les corps gras pâteux susceptibles d'être utilisés dans la phase grasse de la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

On peut encore citer les polyéthers liposolubles résultants de la polyéthérification entre un ou plusieurs diols en C2-C100, de préférence C2-C50. Par exemple, il peut s'agir de l'éther de dodécanediol et de polyéthylène glycol (45OE) vendu sous la dénomination ELFACOS ST 9 par la société AKZO NOBEL.

Comme corps gras pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société DOW CORNING sous les noms commerciaux de DC2503^{®} et DC25514^{®} et leurs mélanges.

Le corps gras pâteux peut être présent dans la composition en une teneur allant de 0,01 à 20%, en particulier de 3 à 15 %, en particulier de 5 à 10 % en poids par rapport au poids total de la composition.

### Phase aqueuse

La composition selon l'invention peut comprendre une phase aqueuse.

La composition selon l'invention peut comprendre de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition selon l'invention, et notamment la phase aqueuse, peut également comprendre des solvants organiques miscibles à l'eau (à température ambiante - 25 °C) comme par exemple les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur allant 0,5% à 85% en poids, par rapport au poids total de la composition, de préférence allant de 5% à 75% en poids, et préférentiellement allant de 10% à 70% en poids.

De préférence, l'eau peut être présente dans la composition selon l'invention en une teneur allant 0,5% à 85% en poids, par rapport au poids total de la composition, de préférence allant de 5% à 75% en poids, et préférentiellement allant de 10% à 70% en poids.

### LES MATIERES COLORANTES

Selon un mode de réalisation particulier de l'invention, la composition comprend au moins une matière colorante.

On entend par matière colorante au sens de la présente invention, un composé susceptible de produire un effet optique coloré lorsqu'il est formulé en quantité suffisante dans un milieu cosmétique approprié.

La matière colorante peut être notamment choisie parmi les pigments, les nacres, les paillettes, les colorants liposolubles, les colorants hydrosolubles, et leurs mélanges.

Par « pigments », il faut comprendre des particules blanches ou colorées, minérales
ou organiques, insolubles dans la phase organique liquide, destinées à colorer et/ou opacifier la composition.
Par « nacres », il faut comprendre des particules irisées, notamment produites par certains mollusques dans leur coquille ou bien synthétisées, insolubles dans le milieu de la composition.
Par « colorants », il faut comprendre des composés généralement organiques solubles dans les corps gras comme les huiles ou dans une phase aqueuse.

Les matières colorantes peuvent être présentes en une teneur allant de 0,0001 % à 60 % en poids, par rapport au poids total de la composition, de préférence allant de 0,1 % à 20 % en poids, et préférentiellement allant de 1 à 15 % en poids.

Selon un mode de réalisation de l'invention, la matière colorante comprend au moins un pigment.

Les pigments peuvent être choisis parmi les pigments minéraux, les pigments organiques, et les pigments composites (c'est-à-dire des pigments à base de matériaux minéraux et/ou organiques).

Par pigments, il faut comprendre des particules de toute forme, dotées d'un effet optique, minérales ou de synthèse, insolubles dans le milieu de la composition quelle que soit la température à laquelle la composition est fabriquée.

Les pigments peuvent être choisis parmi les pigments monochromes, les laques, les nacres, les pigments à effet optiques, comme les pigments réfléchissants et les pigments goniochromatiques.

Les pigments minéraux peuvent être choisis parmi les pigments d'oxyde métallique, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, les oxydes de fer, le dioxyde de titane, les oxydes de zinc, l'oxyde de cérium, l'oxyde de zirconium, l'oxyde de chrome ; le violet de manganèse, le bleu de prusse, le bleu outremer, le bleu ferrique, l'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

Les particules organiques destinées à être enrobées peuvent être par exemple :
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique ;
- les pigments mélaniques.

Parmi les pigments organiques, on peut citer les D&C Blue n°4, D&C Brown n° 1, D&C Green n°5, D&C Green n°6, D&C Orange n°4 , D&C Orange n°5 , D&C Orange n°10, D&C Orange n°11 , D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, D&C Violet n°2, D&C Yellow n°7, D&C Yellow n°8, D&C Yellow n°10, D&C Yellow n°11, FD&C Blue n° 1, FD&C Green n°3, FD&C Red n°40 , FD&C Yellow n°5, FD&C Yellow n°6.

La laque organique peut aussi être supportée par tout support compatible tel qu'un support minéral comme les particules d'alumine, d'argile, de zircone ou d'oxydes métalliques, notamment d'oxyde de zinc ou d'oxyde de titane, de talc, de carbonate de calcium, de sulfate de baryum. De préférence, le support minéral est choisi parmi l'alumine, l'oxyde de titane et le sulfate de barium.
La laque organique peut également être supportée par un support tel que la colophane ou le benzoate d'aluminium.

Parmi les laques organiques, on peut en particulier citer celles connues sous les dénominations suivantes : D & C Red Aluminium lake ; D & C Blue Aluminium lake ;D & C Green Aluminium lake ; D & C Orange Aluminium lake ; D & C Yellow Aluminium lake

Les composés chimiques correspondant à chacun des pigments organiques cités précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande à titre de référence.

Les pigments mélaniques utilisables selon l'invention sont en particulier :
- les pigments mélaniques dérivés de sources naturelles ou synthétiques et qui peuvent être obtenus : (A) par oxydation d'au moins un composé indolique ou indolinique, ou (B) par polymérisation, oxydante ou enzymatique, de précurseurs mélaniques, ou (C) par extraction de la mélanine à partir de substances en contenant,
ou (D) par culture de microorganismes. De tels pigments mélaniques sont notamment décrits dans les documents EP-A-518773, WO-A-93/13744 et WO-A-93/13745.

Les pigments peuvent être présents dans la composition selon l'invention en une teneur allant de 0,01 % à 25 % en poids, par rapport au poids total de la composition, et de préférence allant de 1 à 12 % en poids, et préférentiellement allant de 3 à 8 % en poids.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les colorants hydrosolubles sont par exemple le jus de betterave, le bleu de méthylène, le caramel.

### Charges additionnelles

La composition selon l'invention peut comprendre des charges additionnelles différentes des élastomères et des particules concaves décrites précédemment.

Par charges, il faut comprendre des particules de toute forme, incolores ou blanches, minérales ou de synthèse, insolubles dans le milieu de la composition quelle que soit la température à laquelle la composition est fabriquée.

Les charges additionnelles peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®), les poudres de poly-β-alanine, les poudres de polyéthylène, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyméthacrylate de méthyle, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Les charges peuvent être présentes dans la composition en une teneur allant de 0,1 % à 80 % en poids, par rapport au poids total de la composition, de préférence allant de 1 % à 25 % en poids, et préférentiellement allant de 3 % à 15 % en poids.

### Additifs

La composition selon l'invention peut comprendre au moins un autre ingrédient cosmétique usuel pouvant être choisi notamment parmi les agents gélifiants et/ou épaississants hydrophiles ou lipophiles, les tensioactifs, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-pollution ou anti-radicaux libres, les sequestrants, les agents filmogènes, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

### GALENIQUES

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte. La composition peut être anhydre, par exemple il peut s'agir d'un stick ou d'une pâte anhydre. La composition peut être une composition non rincée.

Selon un mode préféré de réalisation, la composition selon l'invention se présente sous la forme d'une composition anhydre.

Selon un mode plus préféré de réalisation, la composition selon l'invention se présente sous la forme d'une poudre compacte.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

L'invention est illustrée plus en détails par les exemples décrits ci-après.

### Exemples comparatifs 1 à 14 :

On a étudié l'influence de différentes charges sur la viscosité de mélanges d'huile et d'élastomère de silicone non sphérique. Les compositions testées sont décrites dans le tableau ci-après.

Les compositions ont été préparées par mélange au Rayneri pendant 30 minutes. La viscosité de chaque composition a été effectuée à 25°C à l'aide d'un viscosimètre Brookfield, équipé d'un mobile 2, 3 ou 4 suivant les échantillons et à une vitesse de 30 tours par minutes.

Les teneurs des ingrédients sont exprimées en pourcentage en poids par rapport au poids total de la composition.

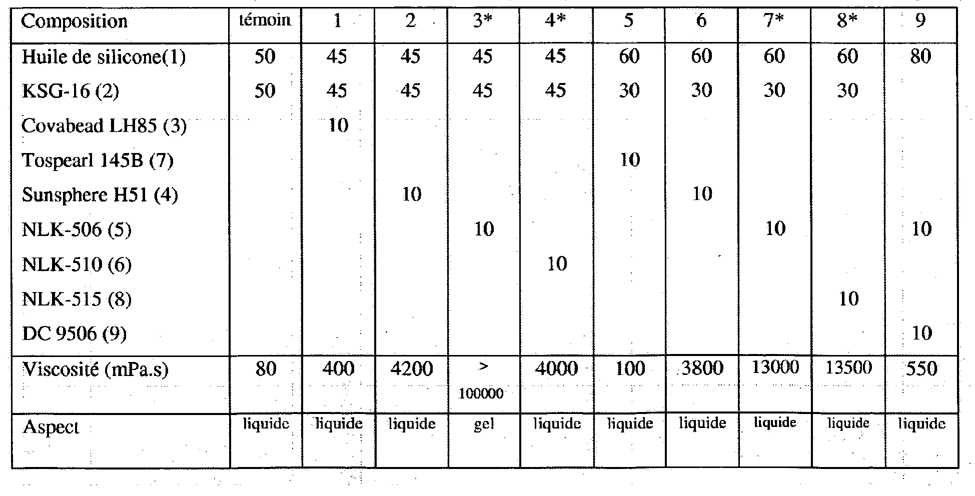

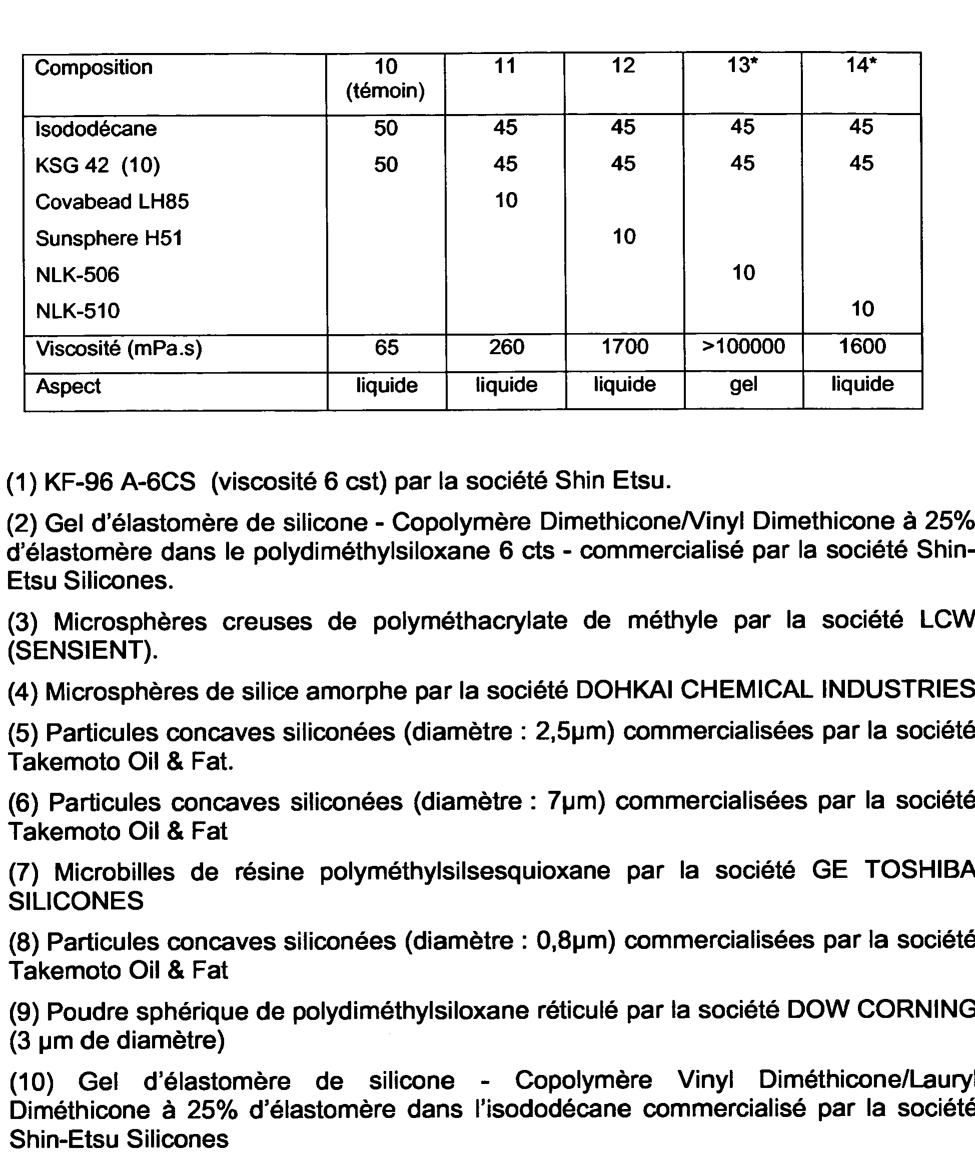

Les compositions témoin ne comprennent pas de charges.

Les compositions 3, 4, 7, 8 selon l'invention ont une viscosité supérieure respectivement aux compositions 1, 2, 5, 6 et 9, ainsi qu'à la composition témoin.

De même, les compositions 13 et 14 ont une viscosité supérieure respectivement aux compositions 11, 12 et à la composition témoin 10.

Les compositions 3, 7, 8 d'un part et 13 d'autre part, correspondent à un mode préféré de réalisation (les particules concaves siliconées ont un diamètre inférieur ou égal à 5 µm) et présentent une viscosité supérieure respectivement aux compositions 4, et 14 également selon l'invention.

### Exemple 15 :

On a préparé un fond de teint anhydre ayant la composition suivante :

| | |
|---|---|
| KSG 42⁽¹⁾ | 20 % |
| NLK-506⁽²⁾ | 10 % |
| Isononyl Isonanoate | 20 % |
| Isododecane | 15 % |
| Gel de Bentone | 10 % |
| Plastic Powder D-400⁽³⁾ | 10 % |
| Talc | 5 % |
| Dioxyde de Titane | 7 % |
| Oxyde de fer | 3 % |

| | |
|---|---|
| (Les teneurs sont exprimées en pourcentage en poids par rapport au poids total de la composition) ⁽¹⁾ Gel d'élastomère de silicone - Copolymère Vinyl Diméthicone/Lauryl Diméthicone à 25% d'élastomère dans l'isododécane commercialisé par la société Shin-Etsu Silicones ⁽²⁾ Particules concaves siliconées (diamètre : 2,5µm) commercialisées par la société Takemoto Oil & Fat. ⁽³⁾ Particules de polyuréthane sphérique commercialisées par Toshiki Pigment | |

Ce fond de teint s'applique aisément sur la peau, avec un bon glissant et une bonne douceur au toucher. Le maquillage obtenu est confortable et ne tiraille pas la peau.

### Exemple 16 :

On a préparé un fond de teint sous forme émulsion eau/huile ayant la composition suivante :

| | |
|---|---|
| NLK 506 ⁽¹⁾ | 8 % |
| KSG-210 ⁽²⁾ | 10 % |
| KSG-16 ⁽³⁾ | 5 % |
| KF-6017 ⁽⁴⁾ | 2 % |
| Cyclopentasiloxane | 15 % |
| Phenyl Trimethicone | 10% |
| Dioxyde de Titane | 7 % |
| Oxyde de fer | 3 % |
| Sulfate de magnésium | 1 % |
| Butylene Glycol | 9 % |
| Eau | qsp 100 % |

| | |
|---|---|
| ⁽¹⁾ Particules concaves siliconées (diamètre : 2,5µm) commercialisées par la société Takemoto Oil & Fat. ⁽²⁾ Gel d'élastomère de silicone - copolymère Dimethicone PEG-10/15 à 25% d'élastomère dans le polydiméthylsiloxane 6 cts commercialisé par la société Shin-Etsu Silicones. ⁽³⁾ Gel d'élastomère de silicone - Copolymère Dimethicone/Vinyl Dimethicone à 25% d'élastomère dans le polydiméthylsiloxane 6 cts - commercialisé par la société Shin-Etsu Silicones. ⁽⁴⁾ tensioactif siliconé de la société Shin Etsu. | |

Le fond de teint obtenu présente un bon glissant lors de son application sur la peau et un toucher doux. Le maquillage obtenu est confortable et ne tiraille pas la peau.

### Exemple 17 :

On a préparé un fond de teint sous forme de poudre compacte ayant la composition suivante :

| | |
|---|---|
| NLK 506 ⁽¹⁾ | 3% |
| KSG 16 ⁽²⁾ | 3% |
| Tospearl 145B ⁽³⁾ | 3 % |
| Talc | 30 % |
| Sericite | 37% |
| Mica | 5% |
| Dioxyde de Titane | 4 % |
| Oxyde de fer | 2 % |
| Amyhope LL ⁽⁴⁾ | 5 % |
| Methoxycinnamate de 2-ethyl-hexyl | 5 % |
| Isononyl Isonanoate | 3 % |

| | |
|---|---|
| ⁽¹⁾ Particules concaves siliconées (diamètre : 2,5µm) commercialisées par la société Takemoto Oil & Fat ⁽²⁾ Gel d'élastomère de silicone - Copolymère DimethiconeNinyl Dimethicone à 25% d'élastomère dans le polydiméthylsiloxane 6 cts - commercialisé par la société Shin-Etsu Silicones. ⁽³⁾ Micro-billes de résine de polyméthylsilsesquioxane vendues par la Société GE Toshiba Silicones. ⁽⁴⁾ Particules de Lauroyl Lysine commercialisées par Ajinomoto. | |

La composition a été préparée en mélangeant l'ensemble des poudres puis en y ajoutant le liant (huiles+KSG16+NLK-506). Le mélange étant ensuite broyé et tamisé jusqu'à obtention d'un mélange homogène. Ce mélange est placé dans une coupelle puis pressé sous une pression de 2 MPa.

On a ainsi obtenu une poudre compacte qui se délite facilement à l'aide d'une éponge et la poudre prélevée est non collante, glisse et s'étale facilement sur la peau.

### Exemple 18 :

On a préparé un stick de rouge à lèvres ayant la composition suivante :

| | |
|---|---|
| Cire de polyéthylène (Polywax 500 de la société Bareco) | 10 g |
| Ozokérite | 5 g |
| Cire microcristalline | 2 g |
| Octyl-2 dodécanol | 20 g |
| Malate de diisostéaryle | 10g |
| Triglycéride d'acides caprique/caprylique | |
| (Myritol 318 de la société Cognis) | 30 g |
| Pigments | 7 g |
| KSG-16 (1) | 10g |
| NLK-506 (2) | 6 g |

| | |
|---|---|
| ⁽¹⁾ Gel d'élastomère de silicone - Copolymère Dimethicone/Vinyl Dimethicone à 25% d'élastomère dans le polydiméthylsiloxane 6 cts - commercialisé par la société Shin-Etsu Silicones. (2) Particules concaves siliconées (diamètre : 2,5µm) commercialisées par la société Takemoto Oil & Fat | |

On broie les pigments en présence d'une partie des huiles. Le restant des huiles est mélangé avec les cires et chauffé à environ 90 °C ; après homogénéisation on ajoute les pigments broyés. On ajoute ensuite les particules NLK-506 et le gel de silicone en dernier.
Le mélange est ensuite coulé dans des moules puis après solidification, les bâtons sont démoulés et conditionnés dans un étui.

Le stick glisse bien sur les lèvres et procure après application un film de rouge à lèvres brillant, confortable, non desséchant.

### Exemple 19 :

On a préparé un fond de teint anhydre ayant la composition suivante :

| | |
|---|---|
| KSG 42⁽¹⁾ | 20 % |
| NLK-515⁽²⁾ | 8% |
| Isononyl Isonanoate | 22 % |
| Isododécane | 15 % |
| Gel de Bentone | 10 % |
| Plastic Powder D-400⁽³⁾ | 10 % |
| Talc | 5% |
| Dioxyde de Titane | 7 % |
| Oxyde de fer | 3% |

| | |
|---|---|
| ⁽¹⁾ Gel d'élastomère de silicone - Copolymère Vinyl Diméthicone/Lauryl Diméthicone à 25% d'élastomère dans l'isododécane commercialisé par la société Shin-Etsu Silicones ⁽²⁾ Particules concaves siliconées (diamètre : 0,8µm) commercialisées par la société Takemoto Oil & Fat. ⁽³⁾ Particules de polyuréthane sphérique commercialisées par Toshiki Pigment | |

Ce fond de teint s'applique aisément sur la peau, avec un bon glissant et une bonne douceur au toucher. Le maquillage obtenu est confortable et ne tiraille pas la peau.

### Exemple 20 :

On a préparé un fond de teint anhydre ayant la composition suivante :

| | |
|---|---|
| KSG 42⁽¹⁾ | 20 % |
| NLK-510⁽²⁾ | 12 % |
| Isononyl Isonanoate | 18 % |
| Isododécane | 15 % |
| Gel de Bentone | 10 % |
| Plastic Powder D-400⁽³⁾ | 10 % |
| Talc | 5 % |
| Dioxyde de Titane | 7 % |
| Oxyde de fer | 3 % |

| | |
|---|---|
| ⁽¹⁾ Gel d'élastomère de silicone - Copolymère Vinyl Diméthicone/Lauryl Diméthicone à 25% d'élastomère dans l'isododécane commercialisé par la société Shin-Etsu Silicones ⁽²⁾ Particules concaves siliconées (diamètre : 7 µm) commercialisées par la société Takemoto Oil & Fat. ⁽³⁾ Particules de polyuréthane sphérique commercialisées par Toshiki Pigment | |

Ce fond de teint s'applique aisément sur la peau, avec un bon glissant et une bonne douceur au toucher. Le maquillage obtenu est confortable et ne tiraille pas la peau.

## Revendications

1. Composition comprenant des particules concaves en un matériau siliconé, notamment sous forme de portions de sphères creuses, et une phase grasse comprenant au moins une huile et au moins un élastomère de silicone non sphérique.

2. Composition selon la revendication précédente, **caractérisée par le fait que** lesdites particules concaves siliconées ont un diamètre moyen inférieur ou égal à 5 µm.

3. Composition selon la revendication précédente, **caractérisée par le fait que** lesdites particules concaves siliconées ont un diamètre moyen allant de 0,1 µm à 5µm, préférentiellement allant de 0,2 à 5µm, plus préférentiellement allant de 0,5 à 4µm et de préférence encore allant de 0,5 à 3µm.

4. Composition selon l'une quelconque des revendications précédentes, **caractérisée par le fait que** les particules concaves siliconées sont sous forme de portions de sphères creuses ayant une section transversale en forme de fer à cheval ou d'arceau.

5. Composition selon l'une quelconque des revendications précédentes, **caractérisée par le fait que** le matériau siliconé est un polysiloxane réticulé de structure tridimensionnelle comprenant, ou constitué de, des motifs de formule (I): SiO₂ et de formule (II) : R¹SiO_{1,5}
dans lesquelles R¹ désigne un groupe organique ayant un atome de carbone directement relié à l'atome de silicium.

6. Composition selon la revendication précédente, **caractérisée par le fait que** le groupe organique est un groupe organique réactif, un groupe organique non réactif, et de préférence un groupe organique non réactif.

7. Composition selon la revendication précédente, **caractérisée par le fait que** le groupe organique non réactif peut être un groupe alkyle en C₁-C₄, ou un groupe phényle.

8. Composition selon la revendication 6 ou 7, **caractérisée par le fait que** le groupe organique non réactif est un groupe méthyle.

9. Composition selon la revendication 6, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

10. Composition selon la revendication 6 ou 9, **caractérisée par le fait que** le groupe organique réactif est choisi parmi un groupe époxy, un groupe (méth)acryloxy, un groupe alkényle, un groupe mercaptoalkyle, aminoalkyle.

11. Composition selon la revendication 5, **caractérisée par le fait que** R¹ désigne un groupe méthyle.

12. Composition selon l'une quelconque des revendications 5 à 11, **caractérisée par le fait que** le matériau siliconé comprend les motifs (I) et (II) selon un rapport molaire motif (I) / motif (II) allant de 30/70 à 50/50, de préférence allant de 35/65 à 45/55.

13. Composition selon l'une quelconque des revendications précédentes,
**caractérisée par** le fait les particules concaves en matériau siliconé sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

14. Composition selon la revendication précédente, **caractérisée par le fait que** dans l'étape (a), le rapport molaire du composé (III) au composé (IV) va de 30/70 à 50/50, de préférence va de 35/65 à 45/45, et préférentiellement est de 40/60.

15. Composition selon la revendication 13 ou 14, **caractérisée par le fait que** le rapport en poids de l'eau au total des composés (III) et (IV) va de 10/90 à 70/30 dans l'étape (a).

16. Composition selon la revendication 13, **caractérisée par le fait que** le groupe organique est un groupe organique réactif, un groupe organique non réactif, et de préférence un groupe organique réactif.

17. Composition selon la revendication précédente, **caractérisée par le fait que** le groupe organique non réactif peut être un groupe alkyle en C₁-C₄, ou un groupe phényle.

18. Composition selon la revendication 16, **caractérisée par le fait que** le groupe organique non réactif est un groupe méthyle.

19. Composition selon la revendication 16, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

20. Composition selon la revendication 16 ou 19, **caractérisée par le fait que** le groupe organique réactif est choisi parmi un groupe époxy, un groupe (méth)acryloxy, un groupe alkényle, un groupe mercaptoalkyle, aminoalkyle.

21. Composition selon la revendication 13, **caractérisée par le fait que** R¹ désigne un groupe méthyle.

22. Composition selon l'une quelconque des revendications 13 à 21, **caractérisée par le fait que** les catalyseurs d'hydrolyse et de polymérisation sont indépendamment choisis parmi l'hydroxyde de sodium, l'hydroxyde de potassium, le carbonate de sodium, l'hydrogénocarbonate de sodium, l'ammoniaque, la triméthylamine, la triéthylamine, l'hydroxyde de tétraméthylammonium, l'acide citrique, l'acide acétique, l'acide méthanesulfonique, l'acide p-toluène sulfonique, l'acide dodécylbenzènesulfonique, l'acide dodécylsulfonique , l'acide chlorhydrique, l'acide sulfurique, l'acide phosphorique.

23. Composition selon l'une quelconque des revendications précédentes, **caractérisée par le fait que** les particules concaves sont formées (en coupe) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

24. Composition selon l'une quelconque des revendications précédentes, **caractérisée par le fait que** les particules concaves siliconées sont présentes en une teneur allant de 0,01 % à 50 % en poids, par rapport au poids total de la composition, de préférence allant de 0,1 % à 30 % en poids, et préférentiellement allant de 1 % à 15 % en poids.

25. Composition selon l'une quelconque des revendications précédentes, **caractérisée en ce que** l'élastomère de silicone non sphérique est choisi parmi ceux obtenus :
- par réaction d'addition réticulation de diorganosiloxane contenant au moins un hydrogène lié au silicium et d'un polyoxyalkylène ayant au moins deux groupements à insaturation éthylénique;
- par réaction d'addition réticulation de diorganosiloxane contenant au moins un hydrogène lié au silicium et de composés polyglycérolés ayant des groupements à insaturation éthylénique, notamment en présence de catalyseur platine ;
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

26. Composition selon la revendication précédente, **caractérisée en ce que** l'élastomère de silicone non sphérique est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

27. Composition selon l'une quelconque des revendications 25 ou 26, **caractérisée en ce que** l'élastomère de silicone non sphérique est obtenu par réaction de diméthylpolysiloxane à terminaisons diméthylvinylsiloxy et de méthylhydrogénopolysiloxane à terminaisons triméthylsiloxy, en présence de catalyseur platine.

28. Composition selon l'une quelconque des revendications précédentes, **caractérisée en ce qu'**elle comprend une huile volatile.

29. Composition selon l'une quelconque des revendications précédentes, **caractérisée en ce qu'**elle comprend une huile non volatile.

30. Composition selon l'une quelconque des revendications 25 à 29, **caractérisée en ce que** l'élastomère de silicone non sphérique est présent en une teneur allant de 0,01 à 30 % en poids, de préférence de 0,1 à 20 % en poids et de façon encore plus préférée de 0,2 à 10 % en poids, par rapport au poids total de ladite composition.

31. Composition selon l'une quelconque des revendications précédentes, **caractérisée en ce que** le rapport massique des particules concaves siliconées sur les élastomères de silicones non sphériques est compris entre 0,1 et 100, de préférence entre 0,2 et 50, de façon encore plus préférentiellement entre 0,5 et 10.

32. Composition selon l'une quelconque des revendications précédentes, **caractérisée en ce que** la phase grasse de ladite composition comprend un corps gras choisi parmi les cires, les corps gras pâteux, et leurs mélanges.

33. Composition selon l'une quelconque des revendications précédentes, **caractérisée en ce qu'**elle comprend au moins une matière colorante.

34. Composition selon la revendication précédente, **caractérisée en ce que** la matière colorante est choisie parmi les pigments, les nacres, les paillettes, les colorants liposolubles, les colorants hydrosolubles, et leurs mélanges.

35. Composition selon l'une quelconque des revendications précédentes, **caractérisée en ce qu'**elle comprend au moins un ingrédient cosmétique choisi parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-pollution ou anti-radicaux libres, les sequestrants, les agents filmogènes, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

36. Composition cosmétique comprenant :
i) des particules concaves siliconées ayant un diamètre moyen inférieur à 5 µm ;
ii) des particules non sphériques d'au moins un élastomère de silicone ;
iii) au moins une huile ;
le rapport massique entre les particules concaves siliconées et les particules non sphériques d'élastomère siliconé est compris entre 0,1 et 100, de préférence entre 0,2 et 50, de façon encore plus préférentielle entre 0,5 et 10.

37. Procédé de maquillage ou de soin des matières kératiniques comprenant l'application sur lesdites matières kératiniques d'une composition cosmétique selon l'une quelconque des revendications précédentes.

38. Utilisation d'une composition cosmétique selon l'une quelconque des revendications 1 à 35 pour obtenir un dépôt, notamment un maquillage, sur la peau ou les lèvres non desséchant et/ou ne tiraillant pas et/ou confortable.

## Claims

1. Composition comprising concave particles made of a silicone material, especially in the form of portions of hollow spheres, and a fatty phase comprising at least one oil and at least one non-spherical silicone elastomer.

2. Composition according to the preceding claim, **characterized in that** said silicone concave particles have an average diameter of less than or equal to 5 µm.

3. Composition according to the preceding claim, **characterized in that** said silicone concave particles have an average diameter ranging from 0.1 µm to 5 µm, preferably ranging from 0.2 to 5 µm, more preferably ranging from 0.5 to 4 µm and more preferably still ranging from 0.5 to 3 µm.

4. Composition according to any one of the preceding claims, **characterized in that** the silicone concave particles are in the form of hollow sphere portions that have a horseshoe-shaped or arch-shaped transverse cross section.

5. Composition according to any one of the preceding claims, **characterized in that** the silicone material is a crosslinked polysiloxane of three-dimensional structure comprising, or composed of, units of formula (I): SiO₂ and of formula (II): R¹SiO₁.₅ in which R¹ denotes an organic group having a carbon atom directly bonded to the silicon atom.

6. Composition according to the preceding claim, **characterized in that** the organic group is a reactive organic group or a non-reactive organic group, and preferably a non-reactive organic group.

7. Composition according to the preceding claim, **characterized in that** the non-reactive organic group may be a C₁-C₄ alkyl group or a phenyl group.

8. Composition according to Claim 6 or 7, **characterized in that** the non-reactive organic group is a methyl group.

9. Composition according to Claim 6, **characterized in that** the reactive organic group is chosen from an epoxy group, a (meth)acryloxy group, an alkenyl group, a mercaptoalkyl, aminoalkyl or haloalkyl group, a glyceroxy group, a ureido group or a cyano group.

10. Composition according to Claim 6 or 9, **characterized in that** the reactive organic group is chosen from an epoxy group, a (meth)acryloxy group, an alkenyl group, a mercaptoalkyl or aminoalkyl group.

11. Composition according to Claim 5, **characterized in that** R¹ denotes a methyl group.

12. Composition according to any one of Claims 5 to 11, **characterized in that** the silicone material comprises the units (I) and (II) in a unit (I)/unit (II) molar ratio ranging from 30/70 to 50/50, preferably ranging from 35/65 to 45/55.

13. Composition according to any one of the preceding claims, **characterized in that** the concave particles made of a silicone material are capable of being obtained according to a process comprising:
(a) the introduction into an aqueous medium, in the presence of at least one hydrolysis catalyst and optionally at least one surfactant, of a compound (III) of formula SiX₄ and of a compound (IV) of formula RSiY₃, where X and Y denote, independently of one another, a C₁-C₄ alkoxy group, an alkoxyethoxy group containing a C₁-C₄ alkoxy group, a C₂-C₄ acyloxy group, an N,N-dialkylamino group containing a C₁-C₄ alkyl group, a hydroxyl group, a halogen atom or a hydrogen atom, and R denotes an organic group having a carbon atom directly bonded to the silicon atom; and
(b) bringing the mixture resulting from step (a) into contact with an aqueous solution containing at least one polymerization catalyst and optionally at least one surfactant, at a temperature between 30 and 85°C, for at least two hours.

14. Composition according to the preceding claim, **characterized in that** in step (a), the molar ratio of the compound (III) to the compound (IV) ranges from 30/70 to 50/50, preferably ranges from 35/65 to 45/45, and preferably is 40/60.

15. Composition according to Claim 13 or 14, **characterized in that** the weight ratio of water to the total of compounds (III) and (IV) ranges from 10/90 to 70/30 in step (a).

16. Composition according to Claim 13, **characterized in that** the organic group is a reactive organic group or a non-reactive organic group, and preferably a reactive organic group.

17. Composition according to the preceding claim, **characterized in that** the non-reactive organic group may be a C₁-C₄ alkyl group or a phenyl group.

18. Composition according to Claim 16, **characterized in that** the non-reactive organic group is a methyl group.

19. Composition according to Claim 16, **characterized in that** the reactive organic group is chosen from an epoxy group, a (meth)acryloxy group, an alkenyl group, a mercaptoalkyl, aminoalkyl or haloalkyl group, a glyceroxy group, a ureido group or a cyano group.

20. Composition according to Claim 16 or 19, **characterized in that** the reactive organic group is chosen from an epoxy group, a (meth)acryloxy group, an alkenyl group, a mercaptoalkyl or aminoalkyl group.

21. Composition according to Claim 13, **characterized in that** R¹ denotes a methyl group.

22. Composition according to any one of Claims 13 to 21, **characterized in that** the hydrolysis and polymerization catalysts are independently chosen from sodium hydroxide, potassium hydroxide, sodium carbonate, sodium hydrogencarbonate, ammonium hydroxide, trimethylamine, triethylamine, tetramethylammonium hydroxide, citric acid, acetic acid, methanesulphonic acid, p-toluenesulphonic acid, dodecylbenzenesulphonic acid, dodecylsulphonic acid, hydrochloric acid, sulphuric acid or phosphoric acid.

23. Composition according to any one of the preceding claims, **characterized in that** the concave particles are formed (in cross section) from a small inner arc (11), from a large outer arc (21) and from segments (31) which connect the ends of the respective arcs, the width (W1) between the two ends of the small inner arc (11) ranging from 0.01 to 8 µm, preferably from 0.02 to 6 µm on average, the width (W2) between the two ends of the large outer arc (21) ranging from 0.05 to 10 µm, preferably from 0.06 to 8 µm on average and the height (H) of the large outer arc (21) ranging from 0.015 to 8 µm, preferably from 0.03 to 6 µm on average.

24. Composition according to any one of the preceding claims, **characterized in that** the silicone concave particles are present in an amount ranging from 0.01% to 50% by weight, relative to the total weight of the composition, preferably ranging from 0.1% to 30% by weight, and preferably ranging from 1% to 15% by weight.

25. Composition according to any one of the preceding claims, **characterized in that** the non-spherical silicone elastomer is chosen from those obtained:
- by crosslinking addition reaction of a diorganosiloxane containing at least one hydrogen bonded to the silicon with a polyoxyalkylene having at least two ethylenically unsaturated groups;
- by crosslinking addition reaction of a diorganosiloxane containing at least one hydrogen bonded to the silicon with polyglycerolated compounds having ethylenically unsaturated groups, especially in the presence of a platinum catalyst;
- by crosslinking addition reaction of a diorganosiloxane containing at least one hydrogen bonded to the silicon with a diorganopolysiloxane having ethylenically unsaturated groups bonded to the silicon;
- by dehydrogenating crosslinking condensation reaction between a diorganopolysiloxane with hydroxyl end groups and a diorganopolysiloxane containing at least one hydrogen bonded to the silicon;
- by crosslinking condensation reaction of a diorganopolysiloxane with hydroxyl end groups and of a hydrolysable organopolysilane;
- by thermal crosslinking of an organopolysiloxane; and
- by crosslinking of an organopolysiloxane via high-energy radiation.

26. Composition according to the preceding claim, **characterized in that** the non-spherical silicone elastomer is obtained by crosslinking addition reaction (A) of a diorganopolysiloxane containing at least two hydrogens each bonded to a silicon, and (B) of a diorganopolysiloxane having at least two ethylenically unsaturated groups bonded to the silicon, especially in the presence (C) of a platinum catalyst.

27. Composition according to either one of Claims 25 and 26, **characterized in that** the non-spherical silicone elastomer is obtained by reaction of a dimethylpolysiloxane having dimethylvinylsiloxy end groups with a methylhydrogenopolysiloxane having trimethylsiloxy end groups in the presence of a platinum catalyst.

28. Composition according to any one of the preceding claims, **characterized in that** it comprises a volatile oil.

29. Composition according to any one of the preceding claims, **characterized in that** it comprises a nonvolatile oil.

30. Composition according to any one of Claims 25 to 29, **characterized in that** the non-spherical silicone elastomer is present in an amount ranging from 0.01 to 30% by weight, preferably from 0.1 to 20% by weight, and more preferably still from 0.2 to 10% by weight, relative to the total weight of said composition.

31. Composition according to any one of the preceding claims, **characterized in that** the weight ratio of the silicone concave particles to the non-spherical silicone elastomers is between 0.1 and 100, preferably between 0.2 and 50, more preferably still between 0.5 and 10.

32. Composition according to any one of the preceding claims, **characterized in that** the fatty phase of said composition comprises a fatty substance chosen from waxes, pasty fatty substances, and mixtures thereof.

33. Composition according to any one of the preceding claims, **characterized in that** it comprises at least one dyestuff.

34. Composition according to the preceding claim, **characterized in that** the dyestuff is chosen from pigments, pearlescent agents, flakes, fat-soluble dyes, water-soluble dyes, and mixtures thereof.

35. Composition according to any one of the preceding claims, **characterized in that** it comprises at least one cosmetic ingredient chosen from hydrophilic or lipophilic gelling agents and/or thickeners, antioxidants, fragrances, preservatives, neutralizers, sunscreens, vitamins, moisturizers, self-tanning compounds, anti-wrinkle active agents, emollients, hydrophilic or lipophilic active agents, agents for combating pollution or free-radical scavengers, sequestrants, film-forming agents, dermo-relaxing agents, calmatives, agents that stimulate the synthesis of dermal or epidermal macromolecules and/or that prevent their degradation, anti-glycation agents, anti-irritants, desquamating agents, depigmenting, anti-pigmenting or pro-pigmenting agents, NO-synthase inhibitors, agents that stimulate the proliferation of fibroblasts or keratinocytes and/or the differentiation of keratinocytes, agents that act on the microcirculation, agents that act on cell energy metabolism, healing agents, and mixtures thereof.

36. Cosmetic composition comprising:
i) silicone concave particles having an average diameter of less than 5 µm;
ii) non-spherical particles of at least one silicone elastomer; and
iii) at least one oil;
the weight ratio of the silicone concave particles to the non-spherical silicone elastomer particles is between 0.1 and 100, preferably between 0.2 and 50, more preferably still between 0.5 and 10.

37. Method for making up or caring for keratin materials comprising the application to said keratin materials of a cosmetic composition according to any one of the preceding claims.

38. Use of a cosmetic composition according to any one of Claims 1 to 35 for obtaining a deposit, especially a make-up, on the skin or lips that is non-drying and/or non-tautening and/or comfortable.

## Patentansprüche

1. Zusammensetzung, die konkave Partikel aus einem Silicon-Material, insbesondere in Form von Teilstücken von Hohlkügelchen, und eine Fettphase umfasst, die mindestens ein Ö1 und mindestens ein nicht kugelförmiges Siliconelastomer umfasst.

2. Zusammensetzung nach dem vorhergehenden Anspruch, **dadurch gekennzeichnet, dass** die konkaven Silicon-Partikel einen mittleren Durchmesser von 5 µm oder darunter haben.

3. Zusammensetzung nach dem vorhergehenden Anspruch, **dadurch gekennzeichnet, dass** die konkaven Silicon-Partikel einen mittleren Durchmesser haben, der im Bereich von 0,1 bis 5 µm, vorzugsweise im Bereich von 0,2 bis 5 µm, noch bevorzugter im Bereich von 0,5 bis 4 µm und noch bevorzugter im Bereich von 0,5 bis 3 µm liegt.

4. Zusammensetzung nach einem der vorhergehenden Ansprüche, **dadurch gekennzeichnet, dass** die konkaven Silicon-Partikel in Form von Teilstücken von Hohlkügelchen vorliegen, die einen Querschnitt in Hufeisenform oder in Form eines Bogens haben.

5. Zusammensetzung nach einem der vorhergehenden Ansprüche, **dadurch gekennzeichnet, dass** das Silicon-Material ein vernetztes Polysiloxan mit dreidimensionaler Struktur ist, das Einheiten der Formel (I): SiO₂ und der Formel (II): R¹SiO₁,₅ umfasst oder das aus diesen Einheiten besteht, in denen R¹ eine organische Gruppe bedeutet, die ein Kohlenstoffatom aufweist, das unmittelbar mit dem Siliciumatom verbunden ist.

6. Zusammensetzung nach dem vorhergehenden Anspruch, **dadurch gekennzeichnet, dass** die organische Gruppe eine reaktive organische Gruppe, eine nicht reaktive organische Gruppe und vorzugsweise eine nicht reaktive organische Gruppe ist.

7. Zusammensetzung nach dem vorhergehenden Anspruch, **dadurch gekennzeichnet, dass** die nicht reaktive organische Gruppe eine (C₁-C₄)-Alkylgruppe oder eine Phenylgruppe sein kann.

8. Zusammensetzung nach Anspruch 6 oder 7, **dadurch gekennzeichnet, dass** die nicht reaktive organische Gruppe eine Methylgruppe ist.

9. Zusammensetzung nach Anspruch 6, **dadurch gekennzeichnet, dass** die reaktive organische Gruppe unter einer Epoxygruppe, einer (Meth)acryloxygruppe, einer Alkenylgruppe, einer Mercaptoalkyl-, Aminoalkyl-, Halogenalkylgruppe, einer Glyceroxygruppe, einer Ureidogruppe, einer Cyanogruppe ausgewählt wird.

10. Zusammensetzung nach Anspruch 6 oder 9, **dadurch gekennzeichnet, dass** die reaktive organische Gruppe unter einer Epoxidgruppe, einer (Meth)aryloxygruppe, einer Alkenylgruppe, einer Mercaptoalkyl-, Aminoalkylgruppe ausgewählt wird.

11. Zusammensetzung nach Anspruch 5, **dadurch gekennzeichnet, dass** R¹ eine Methylgruppe bedeutet.

12. Zusammensetzung nach einem der Ansprüche 5 bis 11 **dadurch gekennzeichnet, dass** das Silicon-Material die Einheiten (I) und (II) in einem Molverhältnis Einheit (I) / Einheit (II) enthält, das im Bereich von 30/70 bis 50/50 und vorzugsweise im Bereich von 35/65 bis 45/55 liegt.

13. Zusammensetzung nach einem der vorhergehenden Ansprüche, **dadurch gekennzeichnet, dass** die konkaven Partikel aus einem Silicon-Material nach einem Verfahren erhältlich sind, das umfasst:
(a) Einbringen in ein wässriges Medium in Gegenwart mindestens eines Hydrolysekatalysators und gegebenenfalls mindestens eines grenzflächenaktiven Stoffs einer Verbindung (III) der Formel SiX₄ und einer Verbindung (IV) der Formel RSiY₃, worin X und Y unabhängig voneinander eine (C₁-C₄)-Alkoxygruppe, eine Alkoxyethoxygruppe, die eine (C₁-C₄)-Alkoxygruppe einschließt, eine (C₂-C₄)-Acyloxygruppe, eine N,N-Dialkylaminogruppe, die eine (C₁-C₄)-Alkylgruppe einschließt, eine Hydroxygruppe, ein Halogenatom oder ein Wasserstoffatom bedeuten und R eine organische Gruppe bedeutet, die ein Kohlenstoffatom umfasst, das unmittelbar mit dem Siliciumatom verbunden ist; und
(b) Inkontaktbringen des aus Schritt (a) resultierenden Gemischs mit einer wässrigen Lösung, die mindestens einen Polymerisationskatalysator und gegebenenfalls mindestens einen grenzflächenaktiven Stoff einschließt, bei einer Temperatur, die im Bereich von 30 bis 85 °C liegt, über einen Zeitraum von mindestens zwei Stunden.

14. Zusammensetzung nach dem vorhergehenden Anspruch, **dadurch** gekenntzeichnet, dass in Schritt (a) das Molverhältnis der Verbindung (III) zu der Verbindung (IV) im Bereich von 30/70 bis 50/50, vorzugsweise im Bereich von 35/65 bis 45/45 liegt und bevorzugt 40/60 ist.

15. Zusammensetzung nach Anspruch 13 oder 14, **dadurch gekennzeichnet, dass** in Schritt (a) das Gewichtsverhältnis des Wassers zur Gesamtmenge der Verbindungen (III) und (IV) im Bereich von 10/90 bis 70/30 liegt.

16. Zusammensetzung nach Anspruch 13, **dadurch gekennzeichnet, dass** die organische Gruppe eine reaktive organische Gruppe, eine nicht reaktive organische Gruppe und vorzugsweise eine reaktive organische Gruppe ist.

17. Zusammensetzung nach dem vorhergehenden Anspruch, **dadurch gekennzeichnet, dass** die nicht reaktive organische Gruppe eine (C₁-C₄)-Alkylgruppe oder eine Phenylgruppe sein kann.

18. Zusammensetzung nach Anspruch 16, **dadurch gekennzeichnet, dass** die nicht reaktive Gruppe eine Methylgruppe ist.

19. Zusammensetzung nach Anspruch 16, **dadurch gekennzeichnet, dass** die reaktive organische Gruppe unter einer Epoxygruppe, einer (Meth)acryloxygruppe, einer Alkenylgurppe, einer Mercaptoalkyl-, Aminoalkyl-, Halogenalkylgruppe, einer Glyceroxygruppe, einer Ureidogruppe, einer Cyanogruppe ausgewählt wird.

20. Zusammensetzung nach Anspruch 16 oder 19, **dadurch gekennzeichnet, dass** die reaktive organische Gruppe unter einer Epoxygruppe, einer (Meth)acryloxygruppe, einer Alkenylgruppe, einer Mercaptoalkyl-, Aminoalkylgruppe ausgewählt wird.

21. Zusammensetzung nach Anspruch 13, **dadurch gekennzeichnet, dass** R¹ eine Methylgruppe bedeutet.

22. Zusammensetzung nach einem der Ansprüche 13 bis 21, **dadurch gekennzeichnet, dass** die Hydrolyse- und Polymerisationskatalysatoren unabhängig unter Natriumhydroxid, Kaliumhydroxid, Natriumcarbonat, Natriumhydrogencarbonat, Ammoniak, Trimethylamin, Triethylamin, Tetramethylammoniumhydroxid, Citronensäure, Essigsäure, Methansulfonsäure, p-Toluolsulfonsäure, Dodecylbenzolsulfonsäure, Dodecylsulfonsäure, Chlorwasserstoffsäure, Schwefelsäure, Phosphorsäure ausgewählt werden.

23. Zusammensetzung nach einem der vorhergehenden Ansprüche, **dadurch gekennzeichnet, dass** die konkaven Partikel (im Schnittbild) aus einem kleinen Innenbogen (11), einem großen Außenbogen (21) und Segmenten (31) gebildet sind, die die Enden der jeweiligen Bögen miteinander verbinden, wobei die Breite (W1) zwischen den beiden Enden des kleinen Innenbogens (11) im Mittel im Bereich von 0,01 bis 8 µm, vorzugsweise im Mittel im Bereich von 0,02 bis 6 µm liegt, die Breite (W2) zwischen den beiden Enden des großen Außenbogens (21) im Mittel im Bereich von 0,05 bis 10 µm, vorzugsweise im Mittel im Bereich von 0,06 bis 8 µm liegt, und die Höhe (H) des großen Außenbogens (21) im Mittel im Bereich von 0,015 bis 8 µm, vorzugsweise im Mittel im Bereich von 0,03 bis 6 µm, liegt.

24. Zusammensetzung nach einem der vorhergehenden Ansprüche, **dadurch gekennzeichnet, dass** die konkaven Silicon-Partikel in einem Anteil enthalten sind, der im Bereich von 0,01 bis 50 Gew.-%, bezogen auf das Gesamtgewicht der Zusammensetzung, vorzugsweise im Bereich von 0,1 bis 30 Gew.-% und bevorzugt im Bereich von 1 bis 15 Gew.-%, liegt.

25. Zusammensetzung nach einem der vorhergehenden Ansprüche, **dadurch gekennzeichnet, dass** das nicht kugelförmige Silicönelastomer unter denjenigen ausgewählt wird, die erhalten werden:
- durch die Vernetzungsadditionsreaktion eines Diorganosiloxans, das mindestens ein Wasserstoffatom enthält, das mit Silicium verbunden ist, mit einem Polyoxyalkylen, das mindestens zwei ethylenisch ungesättigte Gruppen aufweist;
- durch die Vernetzungsadditionsreaktion eines Diorganosiloxans, das mindestens ein Wasserstoffatom enthält, das mit Silicium verbunden ist, mit mehrfach mit Glycerin veretherten Verbindungen, die ethylenisch ungesättigte Gruppen aufweisen, insbesondere in Gegenwart eines Platinkatalysators;
- durch die Vernetzungsadditionsreaktion eines Diorganosiloxans, das mindestens ein Wasserstoffatom enthält, das mit Silicium verbunden ist, mit einem Diorganopolysiloxan, das ethylenisch ungesättigte Gruppen aufweist, die mit Silicium verbunden sind;
- durch die Dehydrierungsvernetzungskondensationsreaktion zwischen einem Diorganopolysiloxan mit endständigen Hydroxygruppen und einem Diorganopolysiloxan, das mindestens ein Wasserstoffatom enthält, das mit Silicium verbunden ist;
- durch die Vernetzungskondensationsreaktion eines Diorganopolysiloxans mit endständigen Hydroxygruppen mit einem hydrolysierbaren Organopolysilan;
- durch die thermische Vernetzung eines Organopolysiloxans;
- durch die Vernetzung eines Organopolysiloxans durch energiereiche Strahlungen.

26. Zusammensetzung nach dem vorhergehenden Anspruch, **dadurch gekennzeichnet, dass** das nicht kugelförmige Siliconelastomer durch die Vernetzungsadditionsreaktion (A) eines Diorganopolysiloxans, das mindestens zwei Wasserstoffatome enthält, die jeweils mit einem Silicium verbunden sind, mit (B) einem Diorganopolysiloxan, das mindestens zwei ethylenisch ungesättigte Gruppen aufweist, die mit dem Silicium verbunden sind, insbesondere in Gegenwart (C) eines Platinkatalysators, erhalten wird.

27. Zusammensetzung nach einem der Ansprüche 25 oder 26, **dadurch gekennzeichnet, dass** das nicht kugelförmige Siliconelastomer durch die Umsetzung eines Dimethylpolysiloxans mit endständigen Dimethylvinylsiloxygruppen mit einem Methylwasserstoffpolysiloxan mit endständigen Trimethylsiloxygruppen in Gegenwart eines Platinkatalysators erhalten wird.

28. Zusammensetzung nach einem der vorhergehenden Ansprüche, **dadurch gekennzeichnet, dass** sie ein flüchtiges Ö1 enthält.

29. Zusammensetzung nach einem der vorhergehenden Ansprüche, **dadurch gekennzeichnet, dass** sie ein nicht flüchtiges Öl enthält.

30. Zusammensetzung nach einem der Ansprüche 25 bis 29, **dadurch gekennzeichnet, dass** das nicht kugelförmige Siliconelastomer in einem Anteil enthalten ist, der im Bereich von 0,01 bis 30 Gew.-%, vorzugsweise 0,1 bis 20 Gew.-% und noch bevorzugter 0,2 bis 10 Gew.-%, bezogen auf das Gesamtgewicht der Zusammensetzung, liegt.

31. Zusammensetzung nach einem der vorhergehenden Ansprüche, **dadurch gekennzeichnet, dass** das Masseverhältnis der konkaven Silicon-Partikel zu den nicht kugelförmigen Siliconelastomeren im Bereich von 0,1 bis 100, vorzugsweise 0,2 bis 50 und noch bevorzugter 0,5 bis 10 liegt.

32. Zusammensetzung nach einem der vorhergehenden Ansprüche, **dadurch gekennzeichnet, dass** die Fettphase der Zusammensetzung eine Fettsubstanz umfasst, die unter den Wachsen, den pastösen Fettsubstanzen und deren Gemischen ausgewählt wird.

33. Zusammensetzung nach einem der vorhergehenden Ansprüche, **dadurch gekennzeichnet, dass** sie mindestens ein Farbmittel enthält.

34. Zusammensetzung nach dem vorhergehenden Anspruch, **dadurch gekennzeichnet, dass** das Farbmittel unter den Pigmenten, den Perlglanzpigmenten, den Plättchen, den fettlöslichen Farbstoffen, den wasserlöslichen Farbstoffen und deren Gemischen ausgewählt wird.

35. Zusammensetzung nach einem der vorhergehenden Ansprüche, **dadurch gekennzeichnet, dass** sie mindestens einen kosmetischen Bestandteil enthält, der unter den hydrophilen oder lipophilen Gelbildner und/oder Verdickungsmitteln, den Antioxidantien, den Parfüms, den Konservierungsmitteln, den Neutralisierungsmitteln, den Sonnenschutzfiltern, den Vitaminen, den Hydratisierungsmitteln, den Selbstbräunungsmitteln, den Antifaltenwirkstoffen, den Emollientien, den hydrophilen oder lipophilen Wirkstoffen, den Mitteln gegen Verschmutzung oder gegen freie Radikale, den Maskierungsmitteln, den Filmbildner, den hautentspannenden Mitteln, den beruhigend wirkenden Mitteln, den Mitteln, die die Synthese von Makromolekülen in der Dermis oder Epidermis stimulieren und/ oder die ihren Abbau verhindern, den Antiglykationsmitteln, den reizlindernden Mitteln, den Abschuppungsmitteln, den Depigmentierungsmitteln, den Antipigmentierungsmitteln oder den die Pigmentierung fördernden Mitteln, den Inhibitoren der NO-Synthase, den Mitteln, die die Vermehrung der Fibroblasten oder Keranozyten anregen und/oder die Differenzierung der Keranozyten anregen, den Mitteln, die auf die Mikrozirkulation einwirken, den Mitteln, die auf den Energiemetabolismus der Zellen einwirken, den Wundheilmitteln und deren Gemischen ausgewählt wird.

36. Kosmetische Zusammensetzung, die enthält:
i) konkave Silicon-Partikel, die einen mittleren Durchmesser von weniger als 5 µm aufweisen;
ii) nicht kugelförmige Partikel aus mindestens einem Siliconelastomer;
iii) mindestens ein Öl;
wobei das Masseverhältnis der konkaven Silicon-Partikel zu den nicht kugelförmigen Partikeln aus Siliconelastomer im Bereich von 0,1 bis 100, vorzugsweise 0,2 bis 50 und noch bevorzugter 0,5 bis 10 liegt.

37. Verfahren zum Schminken oder Pflegen von Keratinmaterialien, das das Auftragen einer kosmetischen Zusammensetzung nach einem der vorhergehenden Verfahren auf die Keratinmaterialien umfasst.

38. Verwendung einer kosmetischen Zusammensetzung nach einem der Ansprüche 1 bis 35 für den Erhalt eines Überzugs, insbesondere einer Schminke, auf der Haut oder den Lippen, der/die nicht austrocknend und nicht ziehend wirkt und komfortabel ist.
